(19) Europäisches Patentamt / European Patent Office / Office européen des brevets

(11) **EP 4 390 953 A1**

(12) **EUROPEAN PATENT APPLICATION**

(43) Date of publication:
**26.06.2024 Bulletin 2024/26**

(21) Application number: **23218097.6**

(22) Date of filing: **19.12.2023**

(51) International Patent Classification (IPC):
**G16H 20/17** (2018.01)   **G16H 40/63** (2018.01)
**A61M 5/172** (2006.01)

(52) Cooperative Patent Classification (CPC):
**G16H 20/17; A61M 5/1723; G16H 40/63**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.12.2022 US 202263476069 P**

(71) Applicant: **Insulet Corporation**
**Acton MA 01720 (US)**

(72) Inventors:
• **LEE, Joon Bok**
 **Acton (US)**
• **CAUSEY, James**
 **Simi Valley (US)**
• **ZADE, Ashutosh**
 **San Diego (US)**
• **O'CONNOR, Jason**
 **Acton (US)**

(74) Representative: **Peterreins Schley**
**Patent- und Rechtsanwälte PartG mbB**
**Hermann-Sack-Straße 3**
**80331 München (DE)**

(54) **AUTOMATIC COMPENSATION FOR VARYING INACCURACY OF SENSOR VALUES USED BY A MEDICAMENT DELIVERY DEVICE**

(57)     Compensation may be provided for the varying accuracy levels of a sensor over time. As a result of the compensation, the medicament delivery device may perform better. The sensor may provide input to a medicament delivery device, and the input may be used to determine a dose of medicament to be delivered to a user by the medicament delivery device. A degree of inaccuracy of sensor values may be determined based on when in the lifetime a sensor value is generated. Glucose level values read by a glucose monitor, such as a CGM, may be directly modified before being used by an automated insulin delivery (AID) control of an insulin delivery device. The compensation for the inaccuracy of the glucose level values from the glucose monitor instead may be achieved by modifying a weight coefficient of a glucose cost component of a cost function in one example.

Figure 1

EP 4 390 953 A1

**Description**

## BACKGROUND

The accuracy of certain on-body medical sensors may vary over their lifetime of use. For example, continuous glucose monitors (CGMs) have an accuracy that may vary over the 10-day lifetime where the CGM is attached to a user and in use detecting glucose levels of the user. For CGMs, the accuracy on the first day of use is substantially lower than the accuracy for subsequent days in the remainder of the lifetime.

Medicament delivery devices, such as insulin delivery devices, rely upon glucose level readings provided by CGMs to determine basal insulin doses for delivery to users. Unfortunately, as mentioned above, the accuracy of the glucose level values from the CGM may vary over time and this variation may not be taken into account by the insulin delivery device. As a result, the doses determined based on the glucose level readings of varying accuracy may be less than ideal and may result in sub-par glucose level control by the insulin delivery device. The risk of erroneous medicament delivery is increased during periods of heightened inaccuracy.

## SUMMARY

In accordance with an inventive facet, a medicament delivery system, in particular medicament delivery device, for delivering a medicament to a user includes a non-transitory computer-readable storage medium storing computer programming instructions and a processor configured to execute the computer programming instructions. Executing the instructions causes the processor to adjust a received sensor value from a sensor for an interval in a period based on an accuracy estimate for the glucose sensor, to use the adjusted received sensor value to determine a dose of medicament to be delivered to the user for the interval, and to cause the determined dose of the medicament to be delivered by the medicament delivery system, in particular medicament delivery device, to the user. The medicament delivery system may be a medicament delivery device or may comprise a medicament delivery device.

The accuracy estimate may be based at least in part on a manufacturer accuracy estimate for the sensor. Executing the computer programming instructions may further cause the processor to determine the accuracy estimate. Executing the computer programming instructions may further cause the processor to determine an accuracy target for the sensor values from the sensor as an average of expected accuracy of the sensor values over a period and determine a robustness factor for the sensor values for the interval in the period as a quotient of the determined accuracy target divided by the expected accuracy of the senor values for the interval of the period. The determined robustness factor may be the accuracy estimate

The adjusting of the received sensor value may entail multiplying the received sensor value by the robustness factor if the robustness factor is less than one. The adjusting of the received sensor value may entail multiplying the received sensor value by one if the robustness factor is greater than or equal to one. The expected accuracy of the sensor values of the period may be a mean absolute relative difference (MARD) value. The sensor may be a glucose monitor, such as a CGM. The medicament may be insulin or include insulin as a component. The glucose monitor (e.g., CGM) has a lifespan, and the period may be the lifespan.

In accordance with another inventive facet, a method comprises adjusting a received sensor value from a sensor for an interval in a period based on an accuracy estimate for the glucose sensor, using the adjusted received sensor value to determine a dose of medicament to be delivered to the user for the interval.

In accordance with another inventive aspect, a medicament delivery system, in particular medicament delivery device, for delivering medicament to a user includes a non-transitory computer-readable storage medium storing computer programming instructions and a processor configured to execute the computer programming instructions. Executing the computer programming instructions causes the processor to determine an accuracy target for the sensor values from a sensor as an average of expected accuracy of the sensor values over a period and to determine a robustness factor for the sensor values for an interval of the period as a quotient of the determined accuracy target divided by an expected accuracy of a sensor value for the interval. Executing the instructions may also cause the processor to adjust a weight coefficient for a cost component of a cost function based on the robustness factor, wherein the cost component reflects a cost of a difference between a target value and a sensor value for a current interval, to use the cost function with the adjusted weight coefficient to determine a dose of the medicament for the current interval, and to cause the determined dose of the medicament to be delivered by the medicament delivery system, in particular medicament delivery device to the user.

The medicament may be insulin or may include insulin as a component. The weight coefficient may be for a glucose cost component that reflects a difference between a target glucose level for the user and a glucose level reading for the current interval. The cost function may include an insulin cost component reflective of how a candidate dose of insulin exceeds a target dose. The period may include multiple days. The interval may be an operational cycle of the medicament delivery system, in particular medicament delivery device, and a dose of the medicament to be delivered may be calculated for each operational cycle while the medicament delivery system, in particular medicament delivery device is in use by the user.

In accordance with another inventive aspect, a method comprises determining an accuracy target for sensor values from a sensor as an average of expected accuracy of the sensor values over a period and determining a robustness factor for the sensor values for an interval of the period as a quotient of the determined accuracy target divided by an expected accuracy of a sensor value for the interval. The method may further comprise adjusting a weight coefficient for a cost component of a cost function based on the robustness factor, wherein the cost component reflects a cost of a difference between a target value and a sensor value for a current interval, and using the cost function with the adjusted weight coefficient to determine a dose of the medicament for the current interval.

In accordance with an additional inventive facet, an on-body glucose sensor that is attached to a user for sensing a glucose level of a user includes a sensing element positioned under skin of the user to sense glucose level values of the user and a filter for filtering the sensed glucose level values to modify the glucose level values based on an accuracy of the glucose sensor or on how long the glucose sensor has been attached to the user to compensate for inaccuracy of the glucose sensor.

The filter may be a dynamic filter that modifies the filtering over time. The filter may be a low pass filter.

In accordance with a further inventive facet, an electronic device includes a non-transitory computer-readable storage medium storing computer programming instructions and a processor configured to execute the computer programming instructions. Executing the instruction causes the processor to determine an accuracy target for sensor values from a sensor over a period as an average of expected accuracy of the sensor values over a period and to determine a robustness factor for the sensor values for an interval in the period as a quotient of the determined accuracy target divided by an expected accuracy of a sensor value for the interval. Executing the instructions may also cause the processor to adjust a sensor value generated by the sensor for the interval based on the determined robustness factor and to forward the adjusted sensor value to a medicament delivery device for use in determining a dose of medicament to be delivered to a user.

The electronic device may be, for example, one of a medicament delivery device, a glucose monitor, or a management device for the medicament delivery device. The medicament may be insulin.

## BRIEF DESCRIPTION OF THE DRAWINGS

Figure 1 depicts a medicament delivery system for delivering medicament to a user in exemplary embodiments.

Figure 2A depicts a flowchart of illustrative steps that may be performed in exemplary embodiments to adjust a sensor value to compensate for inaccuracy.

Figure 2B depicts a flowchart of illustrative steps that may be performed in exemplary embodiments to trigger an adjustment of a sensor value(s) based on time.

Figure 2C depicts a flowchart of illustrative steps that may be performed in exemplary embodiments to trigger an adjustment of a sensor value(s) based on detection of an event.

Figure 3A depicts an example of an insulin delivery device adjusting a glucose level value from a CGM.

Figure 3B depicts an example of a CGM adjusting a glucose level value from a detector of the CGM.

Figure 3C depicts an example of a management device for an insulin delivery device adjusting a glucose level value from a CGM.

Figure 4 depicts a flowchart of illustrative steps that may be performed in exemplary embodiments to adjust a glucose level value originating from a glucose monitor.

Figure 5 depicts a flowchart of illustrative steps that may be performed in exemplary embodiments to determine an accuracy target for a sensor.

Figure 6 depicts a flowchart of illustrative steps that may be performed in exemplary embodiments to determine a robustness factor.

Figure 7 depicts a flowchart of illustrative steps that may be performed in exemplary embodiments to adjust a glucose level value.

Figure 8 depicts a flowchart of illustrative steps that may be performed in exemplary embodiments to use an adjusted weight coefficient in a cost function.

Figure 9 depicts a flowchart of illustrative steps that may be performed in exemplary embodiments to adjust the weight coefficient.

Figure 10 depicts a flowchart of illustrative steps that may be performed in exemplary embodiments to determine an adjustment factor for a weight coefficient.

Figure 11 depicts a diagram of illustrative sources of accuracy estimates in exemplary embodiments.

Figure 12 depicts a flowchart of illustrative steps that may be performed in exemplary embodiments to calculate a MARD value in accordance with a first option.

Figure 13 depicts a flowchart of illustrative steps that may be performed in exemplary embodiments to calculate a MARD value in accordance with a second option.

## DETAILED DESCRIPTION

The exemplary embodiments may compensate for varying accuracy levels of a sensor over at least a portion of the lifetime of the sensor. The sensor may provide input values and age of the values to a medicament delivery device, and the input may be used to determine a dose of medicament to be delivered to a user by the medicament delivery device. The exemplary embodiments may determine or estimate a degree of inaccuracy of sensor values based on when in the lifetime of the sensor a sensor value is generated. As a result of the compensation, the medicament delivery device may perform better. For example, where the medicament delivery device is an insulin delivery device, the insulin delivery device may deliver more accurate insulin delivery amounts based on truer needs of the user and thereby provide better glucose level control for the user as a result of the compensation.

In some exemplary embodiments, glucose level values read by a glucose monitor, such as a CGM, may be directly modified before being used by an automated insulin delivery (AID) control of an insulin delivery device. The modifying compensates for the inaccuracy of the glucose level values provided by the readings and accounts for variability in the accuracy levels of the glucose level values from the glucose monitor over the lifetime of the glucose monitor. The modifying may be performed by the insulin delivery device, the glucose monitor, or a management device for the insulin delivery device in some exemplary embodiments.

In other exemplary embodiments, compensation for the inaccuracy of the glucose level values from the glucose monitor may be achieved by modifying a weight coefficient of a glucose cost component of a cost function. The cost function may be used in determining a basal insulin dose to be delivered to the user by the insulin delivery device. The weight coefficient may be modified to increase the aggressiveness of the AID control in eliminating glucose excursions when the accuracy is higher than average. As a result, glucose level control may be improved.

The discussion below often focuses on the case where the sensor is a CGM and the medicament delivery device is an insulin delivery device. Nevertheless, there are inventive aspects that relate as well to other sensors and to other types of medicament delivery devices.

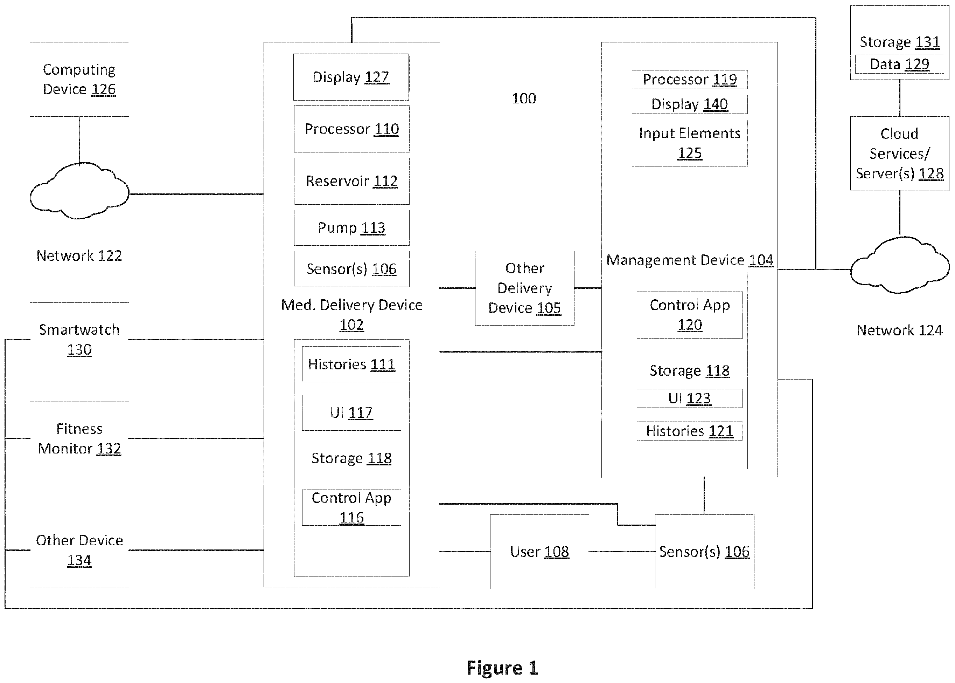

Figure 1 depicts a block diagram of an illustrative medicament delivery system 100 that is suitable for delivering a medicament to a user 108 in accordance with the exemplary embodiments. The medicament delivery system 100 includes a medicament delivery device 102. The medicament delivery device 102 may be a wearable device that is worn on the body of the user 108 or carried by the user. The medicament delivery device 102 may be directly coupled to a user (e.g., directly attached to a body part and/or skin of the user 108 via an adhesive or the like) with no tubes and an infusion location directly under the medicament delivery device 102, or carried by the user (e.g., on a belt or in a pocket) with the medicament delivery device 102 connected to an infusion site where the medicament is injected using a needle and/or cannula. A surface of the medicament delivery device 102 may include an adhesive to facilitate attachment to the user 108.

The medicament delivery device 102 may include a processor 110. The processor 110 may be, for example, a microprocessor, a logic circuit, a field programmable gate array (FPGA), an application specific integrated circuit (ASIC) or a microcontroller. The processor 110 may maintain a date and time as well as other functions (e.g., calculations or the like). The processor 110 may be operable to execute a control application 116 encoded in computer programming instructions stored in the storage 114 that enables the processor 110 to direct operation of the medicament delivery

4

device 102. The control application 116 may be a single program, multiple programs, modules, libraries or the like. The processor 110 also may execute computer programming instructions stored in the storage 114 for a user interface (UI) 117 that may include one or more display screens shown on display 127. The display 127 may display information to the user 108 and, in some instances, may receive input from the user 108, such as when the display 127 is a touchscreen.

The control application 116 may control delivery of a medicament to the user 108 per a control approach like that described herein. This control may be realized as an AID control component. The AID control component may be part of the control application 116 in some embodiments or may be a separate invocable component that is invoked by the control application 116 in other embodiments. The storage 114 may hold histories 111 for a user, such as a history of basal deliveries, a history of bolus deliveries, and/or other histories, such as a meal event history, exercise event history, glucose level history, and/or the like. In addition, the processor 110 may be operable to receive data or information. The storage 114 may include both primary memory and secondary memory. The storage 114 may include random access memory (RAM), read only memory (ROM), optical storage, magnetic storage, removable storage media, solid state storage or the like.

The medicament delivery device 102 may include a tray or cradle and/or one or more housings for housing its various components including a pump 113, a power source (not shown), and a reservoir 112 for storing a medicament for delivery to the user 108. A fluid path to the user 108 may be provided, and the medicament delivery device 102 may expel the medicament from the reservoir 112 to deliver the medicament to the user 108 using the pump 113 via the fluid path. The fluid path may, for example, include tubing coupling the medicament delivery device 102 to the user 108 (e.g., tubing coupling a cannula to the reservoir 112), and may include a conduit to a separate infusion site. The medicament delivery device 102 may have operational cycles, such as every 5 minutes, in which basal doses of medicament are calculated and delivered as needed. These steps are repeated for each cycle.

There may be one or more communications links with one or more devices physically separated from the medicament delivery device 102 including, for example, a management device 104 of the user and/or a caregiver of the user, sensor(s) 106, a smartwatch 130, a fitness monitor 132 and/or another variety of device 134. The communication links may include any wired or wireless communication links operating according to any known communications protocol or standard, such as Bluetooth®, Wi-Fi, a near-field communication standard, a cellular standard, or any other wireless protocol. The embodiments presented herein may also be performed by a plurality of processors for example in a distributed computer system.

The medicament delivery device 102 may interface with a network 122 via a wired or wireless communications link. The network 122 may include a local area network (LAN), a wide area network (WAN) or a combination thereof. A computing device 126 may be interfaced with the network 122, and the computing device may communicate with the medicament delivery device 102.

The medicament delivery system 100 may include one or more sensor(s) 106 for sensing the levels of one or more analytes. The sensor(s) 106 may be coupled to the user 108 by, for example, an adhesive or the like and may provide information or data on one or more medical conditions and/or physical attributes of the user 108. The sensor(s) 106 may be physically separate from the medicament delivery device 102 or may be an integrated component thereof. The sensor(s) 106 may include, for example, glucose monitors, such as CGMs and/or non-invasive glucose monitors. The sensor(s) 106 may include ketone sensors, analyte sensors, heart rate monitors, breathing rate monitors, motion sensors, temperature sensors, perspiration sensors, blood pressure sensors, alcohol sensors, or the like. Some sensors 106 may also detect characteristics of components of the medicament delivery device 102. For instance, the sensors 106 in the medicament delivery device may include any of the foregoing listed sensors, as well as voltage sensors, current sensors, and the like.

The medicament delivery system 100 may or may not also include a management device 104. In some embodiments, no management device is needed as the medicament delivery device 102 may manage itself. The management device 104 may be a special purpose device, such as a dedicated personal diabetes manager (PDM) device. The management device 104 may be a programmed general-purpose device, such as any portable electronic device including, for example, a dedicated controller, such as a processor, a micro-controller, or the like. The management device 104 may be used to program or adjust operation of the medicament delivery device 102 and/or the sensor(s) 106. The management device 104 may be any portable electronic device including, for example, a dedicated device, a smartphone, a smartwatch, or a tablet. In the depicted example, the management device 104 may include a processor 119 and a storage 118. The processor 119 may execute processes to manage a user's glucose levels and to control the delivery of the medicament to the user 108. The medicament delivery device 102 may provide data from the sensors 106 and other data to the management device 104. The data may be stored in the storage 118. The processor 119 may also be operable to execute programming code stored in the storage 118. For example, the storage 118 may be operable to store one or more control applications 120 for execution by the processor 119. The control application 120 may be responsible for controlling the medicament delivery device 102, such as by controlling the AID of insulin to the user 108. The storage 118 may store the control application 120, histories 121 like those described above for the medicament delivery device 102, and other data and/or programs.

A display 140, such as a touchscreen, may be provided for displaying information. The display 140 may display user interface (UI) 123. The display 140 also may be used to receive input, such as when it is a touchscreen. The management device 104 may further include input elements 125, such as a keyboard, button, knobs, or the like, for receiving input form the user 108.

The management device 104 may interface with a network 124, such as a LAN or WAN or combination of such networks, via wired or wireless communication links. The management device 104 may communicate over network 124 with one or more servers or cloud services 128. Data, such as sensor outputs or sensor values, may be sent, in some embodiments, for storage and processing from the medicament delivery device 102 directly to the cloud services/server(s) 128 or instead from the management device 104 to the cloud services/server(s) 128.

Other devices, like smartwatch 130, fitness monitor 132 and device 134 may be part of the medicament delivery system 100. These devices 130, 132 and 134 may communicate with the medicament delivery device 102 and/or management device 104 to receive information and/or issue commands to the medicament delivery device 102. These devices 130, 132 and 134 may execute computer programming instructions to perform some of the control functions otherwise performed by processor 110 or processor 119, such as via control applications 116 and 120. These devices 130, 132 and 134 may include displays for displaying information. The displays may show a user interface for providing input by the user, such as to request a change or pause in dosage, or to request, initiate, or confirm delivery of a bolus of a medicament, or for displaying output, such as a change in dosage (e.g., of a basal delivery amount) as determined by processor 110 or management device 104. These devices 130, 132 and 134 may also have wireless communication connections with the sensor 106 to directly receive analyte measurement data. Another delivery device 105, such as a medicament delivery pen, may be accounted for or may be provided for also delivering medicament to the user 108.

A wide variety of medicaments may be delivered by the medicament delivery device 102 and delivery device 105. The medicament may be insulin for treating diabetes. The medicament may be glucagon for raising a user's glucose level. The medicament may also be a glucagon-like peptide (GLP)-1 receptor agonists for lowering glucose or slowing gastric emptying, thereby delaying spikes in glucose after a meal. Alternatively, the medicament delivered by the medicament delivery device 102 may be one of a pain relief agent, a chemotherapy agent, an antibiotic, a blood thinning agent, a hormone, a blood pressure lowering agent, an antidepressant, an antipsychotic, a statin, an anticoagulant, an anticonvulsant, an antihistamine, an anti-inflammatory, a steroid, an immunosuppressive agent, an antianxiety agent, an antiviral agent, a nutritional supplement or a vitamin. The medicament may be a coformulation of two or more of those medicaments listed above.

The functionality described herein for the exemplary embodiments may be under the control of or performed by the control application 116 of the medicament delivery device 102 or the control application 120 of the management device 104. In some embodiments, the functionality wholly or partially may be under the control of, or performed by, the cloud services/servers 128, the computing device 126 or by the other enumerated devices, including smartwatch 130, fitness monitor 132 or another wearable device 134.

In a closed loop or automated mode, the control application 116, 120 determines the medicament delivery amount for the user 108 on an ongoing basis based on a feedback loop. For an insulin delivery device, the aim of the closed loop mode is to have the user's glucose level at a target glucose level or within a target glucose range.

In the medicament delivery device 102, the automatic determination of basal medicament doses relies upon values from a sensor 106 (e.g., a glucose monitor, like a CGM). Given that there is known to be a time-varying inaccuracy of readings from some sensors like CGMs, steps may be taken to correct for the inaccuracy so that the determination of basal medicament doses is not negatively affected by the inaccuracy. The corrective steps may be taken by the medicament delivery device 102, the sensor 106, or even the management device 104 in exemplary embodiments.

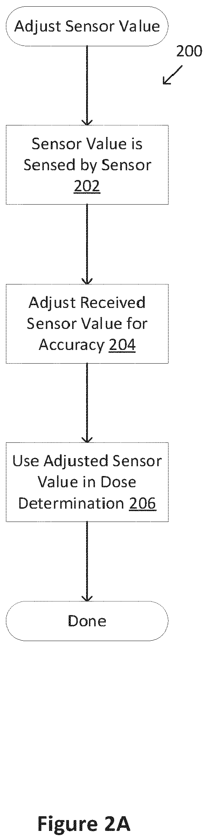

Figure 2A depicts a flowchart 200 of illustrative steps that may be performed in exemplary embodiments to adjust an inaccurate sensor value to compensate for the inaccurate sensor value in determining a medicament dose for a user of a medicament delivery device 102. At 202, a sensor value is detected by the sensor 106. For example, a CGM may detect a glucose level value for the user 108. At 204, the sensor value that was sensed by the sensor 106 is adjusted to compensate for the inaccuracy. For instance, the sensor value may need to be increased or decreased by a correction factor to compensate for the inaccuracy. A safety precaution may be implemented such that a sensor value may only be decreased to compensate for inaccuracy of the sensor 106. Additionally or alternatively, caps may be set such that the sensor value may only be changed by a particular amount, e.g., a particular percentage such as 5%, 10%, 15%, or 20%, to compensate for inaccuracy of sensor 106. At 206, the adjusted sensor value is used in dose determination by the medicament delivery device 102.

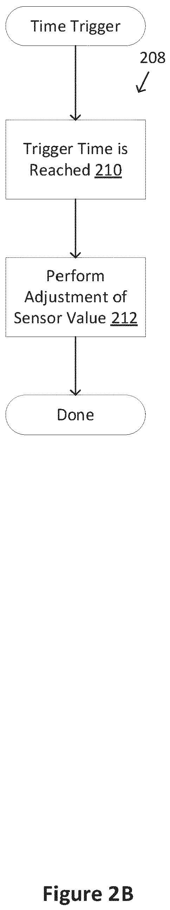

The adjustment in Figure 2A may performed a single time or may be repeated based on triggers. One such trigger is the elapsing of an interval. Figure 2B depicts a flowchart 208 of illustrative steps that may be performed in exemplary embodiments where the trigger is the elapsing of a time interval. At 210, a trigger time is reached. For instance, a trigger time may be reached after a certain amount of time (e.g., 4 hours, 6 hours, 8 hours, 12 hours, or 24 hours) from when the sensor 106 last calibrated itself, or when the user last calibrated sensor 106 (e.g., with a manual finger prick blood glucose check). Additionally, or alternatively, the adjustment may need to be performed every 4 hours. In this example, every four hours a trigger time is reached. Some medicament delivery devices 102 have operational cycles that drive activities. For example, an insulin delivery device may have an operational cycle of 5 minutes. Thus, every 5 minutes, a new cycle begins, a glucose level reading is obtained from a glucose sensor, and a basal insulin dose is determined for delivery for the cycle. Once the trigger time is reached, at 212, the sensor value may be adjusted as detailed in Figure 2A.

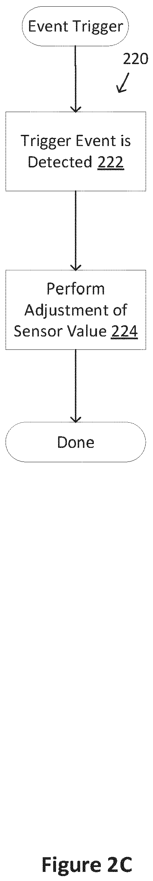

The trigger may also be an event. Figure 2C depicts a flowchart 220 of illustrative steps that may be performed in exemplary embodiments where an event triggers an adjustment of a sensor value. At 220, a trigger event is detected. The trigger event may vary with the type of medicament delivery device and the type of sensor. One example of a trigger event is that the performance of the medicament delivery device is outside a desired range. For instance, the glucose levels for a user 108 may be outside a desired range or a difference between a target glucose level and an actual glucose level may be above a threshold, in particular for an amount of time exceeding a threshold amount of time. Other examples of a trigger event include application, attachment, or initiation of a (new) medicament delivery device or a glucose sensor to a user. Once the trigger event is detected, at 212, the sensor value may be adjusted as detailed in Figure 2A. In some exemplary embodiments, both trigger times and trigger events may be used.

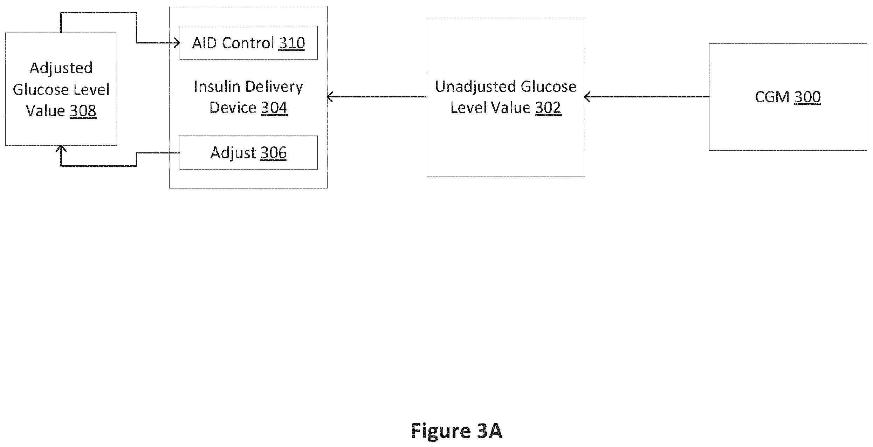

As was mentioned above, the adjustment of the sensor values may be done by different actors. Figure 3A depicts an example where the adjustment is performed by the medicament delivery device. In this example, the medicament delivery device is an insulin delivery device 304, and the sensor is CGM 300. As shown, CGM 300 may produce an unadjusted glucose level value 302. The unadjusted glucose level value 302 may be sent to the insulin delivery device 304, such as through a wireless connection, like a Bluetooth Low Energy (BLE) connection. The insulin delivery device 304 may include an adjust module 306 for adjusting the unadjusted glucose level value. The adjust module 306 may be part of the control application 116 or may be a separate module. The adjust module 306 may contain computer programming instructions for performing the adjustments, as will be detailed below. The adjust module 306 may produce an adjusted glucose level value 308, which is sent to the AID control 310. The AID control 310 may use the adjusted glucose level value 302 to determine a next basal insulin dose for the user 108 and may cause the delivery of the determined basal insulin dose to the user 108. The AID control may be part of control application 116 or 120 or may be invoked by the control application 116 or 120.

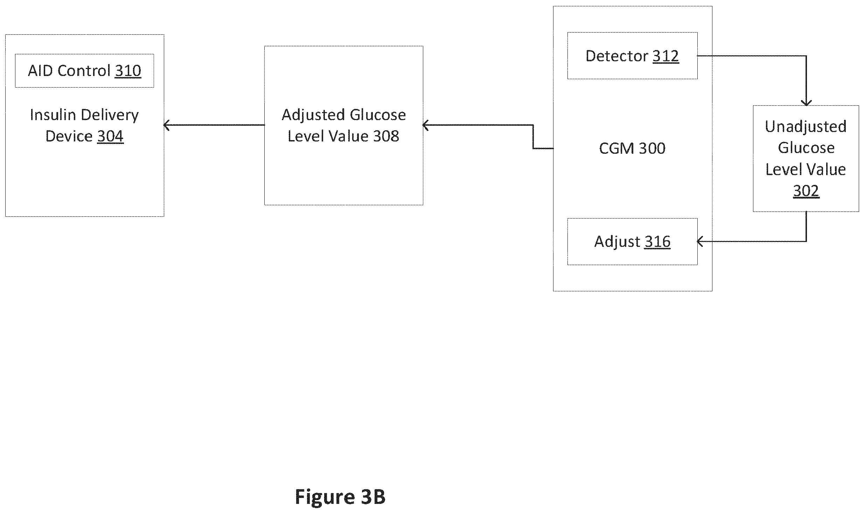

The adjustment of the unadjusted glucose level value may also be performed by the sensor. Figure 3B depicts an example where the sensor is a CGM 300. The CGM 300 has a detector 312 that is positioned under the skin of the user 108 in an interstitial space to detect a glucose level of the user 108. The detector 312 may produce an unadjusted glucose level value 302. The CGM 300 includes an adjust module 316 that may adjust the unadjusted glucose level value 302 to produce an adjusted glucose level value 308. The adjust module 316 may include computer programming instructions that perform the adjustment. In some exemplary embodiments, the adjust module 316 acts as a filter that performs the adjustment. The filter may be a bandpass filter, such as a lowpass filter in some embodiments. The filter may be dynamic. The adjusted glucose level value 308 may be sent to the insulin delivery device 304, such as via a wireless connection. The AID control 310 of the insulin delivery device 304 may use the adjusted glucose level value 308 to determine a next basal insulin dose for the user 108 and may cause the delivery of the determined basal insulin dose to the user 108.

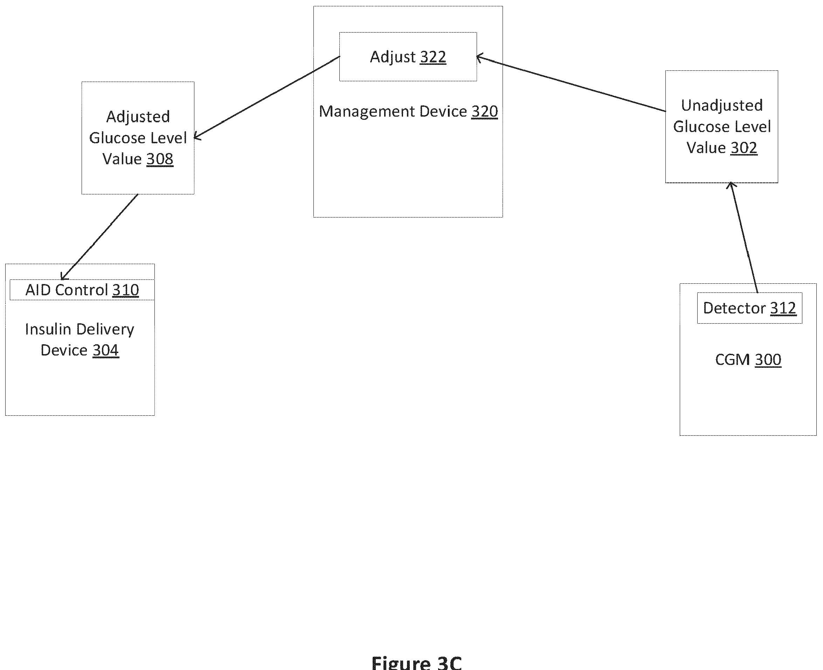

The adjustment may be performed by a management device 104 that manages the medicament delivery device 102. Figure 3C depicts such an arrangement. A sensor 106, like CGM 300, may have a detector 312. The detector 312 may detect an unadjusted glucose level value 302, and the unadjusted glucose level value 302 may be passed to the management device 320 via a wireless connection. The management device 320 may include an adjust module 322 that includes computer programming instructions for adjusting the unadjusted glucose level value 302. The management device 320 may execute the computer programming instructions on processor 119, as described above. The resulting adjusted glucose level values 308 may be passed to the insulin delivery device 304, such as via a wireless connection. The adjusted glucose level value 308 may be used by the AID control 310 in determining a next basal insulin dose, that is ultimately delivered to the user 108.

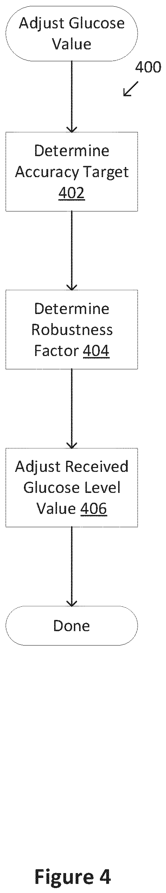

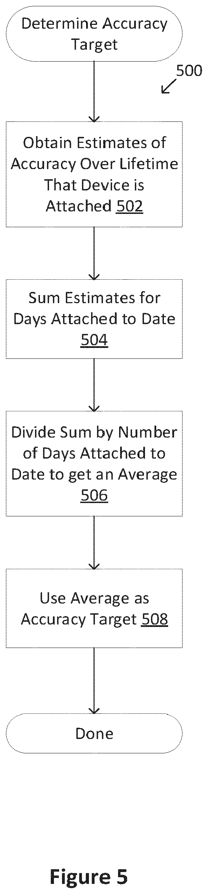

Figure 4 depicts a flowchart 400 of illustrative steps that may be performed in exemplary embodiments to realize the adjustment of an unadjusted glucose level value. At 402, an accuracy target may be determined. Figure 5 depicts a flowchart 500 of illustrative steps that may be performed in exemplary embodiments to determine the accuracy target. First, at 502, estimates of accuracy of the sensor, such as CGM 300, over the lifetime that the device is attached to the user 108, are determined. At 504, these estimates may be summed over the lifetime that the sensor 106 is attached to the user. For a CGM that is attached to the user for 10 days, the sum may be expressed as $\sum_j^k MARD(j)$, where $k$ is the total number of days that the CGM is attached to the user and $j$ is a day index value. $MARD(j)$ is the mean absolute relative difference. Manufacturers publish the $MARD(j)$ values for CGMs for the expected lifetime of attachment of the CGMs. $MARD$ quantifies the mean absolute difference in comparison to the size of the mean. Typical $MARD$ values for a CGM may vary between 9% to 15% or more over the expected time of attachment (e.g., 10 days). The published *MARD* values may be used or other approaches may be used as described below. At 506, the summation may be divided by the total days of attachment, *k*, and at 508, the resulting quotient is used as the accuracy target. Thus, the calculation of the accuracy target may be expressed as:

$$A_{target} = \frac{\sum_{j=2}^{k} MARD(k)}{k} \quad \text{(Equation 1)}$$

where $A_{target}$ is the accuracy target. Hence, in this formulation, $A_{target}$ is the average *MARD* for the period of days from day 2 to day *k,* for example. It should be appreciated that $A_{target}$ may in some instances be determined for periods of minutes, hours or weeks in some embodiments rather than over a period of days.

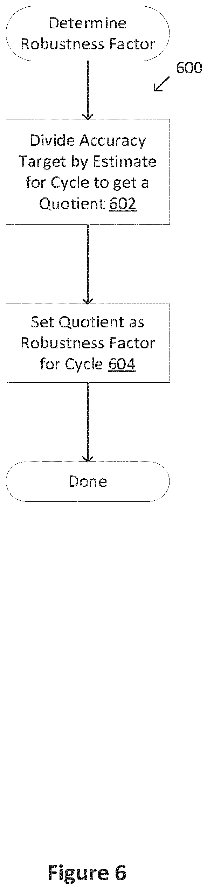

Once the accuracy target is determined at 402, a robustness factor may be determined at 404. The robustness factor is a factor that captures how robust a glucose level values is. Figure 6 depicts a flowchart 600 of illustrative steps that may be performed in exemplary embodiments to determine the robustness factor. The robustness factor may be determined on a cycle-by-cycle basis in some exemplary embodiments. At 602, the accuracy target, which is a daily average in this case, is divided by an accuracy estimate for a current cycle to yield a quotient. If the cycles are 5 minutes in length, there are 288 cycles in a day. Hence, the first cycle of a day may be cycle 1, the second cycle may be cycle 2, and so forth until the last cycle is enumerated as cycle 288. At 604, the quotient is used as the robustness factor for the cycle. An equation for the robustness factor may be expressed as:

$$R_f(t) = \frac{A_{target}}{MARD(t)} \quad \text{(Equation 2)}$$

where $R_f(t)$ is the robustness factor for cycle *t* and *MARD(t)* is the accuracy estimate for cycle *t,* which may be determined by determining what the accuracy estimate is for the day that includes cycle *t*. As can be seen from Equation 2, the robustness factor expresses how much the estimate of accuracy for the current cycle varies from the average expected accuracy as expressed by the accuracy target.

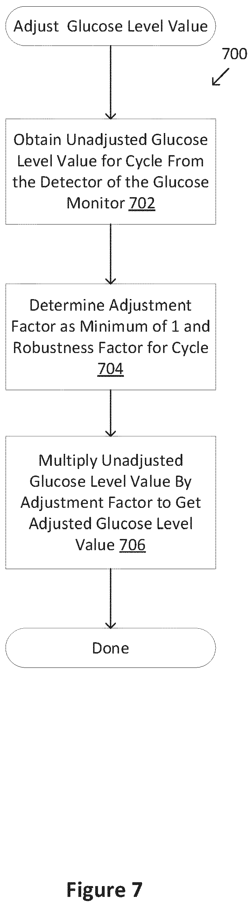

With reference to Figure 4, at 406, the robustness factor may be used to adjust the unadjusted glucose level value. Figure 7 depicts a flowchart 700 of illustrative steps that may be performed in exemplary embodiments to adjust the unadjusted glucose level values using the robustness factor. At 702, the unadjusted glucose level value for the current cycle may be obtained from the detector of the glucose monitor (e.g., CGM). At 704, an adjustments factor may be obtained as the minimum (i.e., smaller) of 1 and the robustness factor for the current cycle. At 706, the unadjusted glucose level value may be multiplied by the adjustment factor to get the adjusted glucose level value. A suitable equation for determining the adjusted glucose level value is:

$$CGM'(t) = CGM(t) \cdot \min\big(1, R_f(t)\big) \quad \text{(Equation 3)}$$

where *CGM(t)* is the unadjusted glucose level value for cycle *t,* $R_f(t)$ is the robustness factor for cycle *t,* min() is a function that returns the minimum of its inputs, and *CGM'(t)* is the adjusted glucose level value for cycle *t*. From the above equation, if the robustness factor $R_f(t)$ is greater than or equal to 1, no adjustments are made. However, if $R_f(t)$ is less than 1, it means that *MARD(t)* is more than $A_{target},$ so the value of *CGM(t)* is decreased. This approach decreases the glucose level value as an adjustment when the unadjusted glucose level values are less accurate and hence, avoids the risk of hypoglycemia due to excessive dosing.

In another alternate approach, the robustness factor may be incorporated into an AID control system parameter of aggressiveness, or the degree to which the AID control system drives toward a desired blood glucose level or range. Specifically, in certain embodiments, the robustness factor can impact the "cost function" of the AID control. A suitable cost function for the exemplary embodiments is:

$$J(t) = Q' \cdot \sum_{i=1}^{M} \big(CGM(i) - target(i)\big)^2 + R \cdot \sum_{i=1}^{N} \big(I_p(i) - I_b(i)\big)^2 \quad \text{(Equation 4)}$$

where *J(t)* is the cost of an insulin dose for cycle *t, target(i)* is a target glucose level for the user for cycle *i,* *CGM(i)* is the expected glucose level of the user at cycle *i, M* is the last cycle in a future time horizon over which the glucose cost is determined, *Q'* is the weight coefficient for the glucose cost component, $I_p(i)$ is the expected insulin dose at cycle *i,* $I_b(i)$

is the expected ideal basal dose for cycle *i* (which may be based on a user's total daily insulin (TDI) value divided by 24 divided by 2), *N* is the cycle number of the last cycle over which the insulin cost is determined, and R is the insulin cost weight coefficient. $Q' \sum_{i=1}^{M} \left( CGM(i) - target(i) \right)^2$ is the glucose cost component of the cost function and

$R \cdot \sum_{i=1}^{N} \left( I_p(i) - I_b(i) \right)^2$ is the insulin cost component of the cost function. Depending on the setup of the cost function, the AID control system may aim to minimize or maximize the cost function. In particular, the AID control system may aim to minimize the cost function disclosed above.

In exemplary embodiments, the robustness factor is used to modify the weight coefficient *Q'* for the glucose cost component and thus influence how greatly glucose excursions are punished in the cost function; that is, the degree of aggressiveness of the control system. A glucose excursion refers to divergences of *CGM(i)* relative to *target(i)*. A larger value of *Q'* magnifies the impact of glucose excursions on the cost and thus makes a dose that would result in glucose excursions less palatable than it otherwise would be (e.g., with a smaller value of *Q '*). Hence, the AID control may be more aggressive in decreasing the excursions (by, for example, delivering smaller or larger doses of insulin). On the other hand, a smaller value of *Q'* decreases the impact of glucose excursions on the cost and makes a dose that would result in glucose excursions more palatable than it otherwise would be (e.g., with a larger value of *Q '*). The AID control may then be less aggressive relative to reducing excursions.

The nexus of the cost function and the weight coefficient to the accuracy is that if the glucose level value from the CGM is known to be more accurate at a cycle *t* (e.g., $R_f(t)$ is greater than 1 because the accuracy target, $A_{target}$ is larger than the MARD value at cycle *t*), then more aggressiveness is acceptable as the CGM value is known to be more accurate. Hence, the original value of the glucose cost weight coefficient Q may be increased. Otherwise (e.g., if $R_f(t)$ is less than 1), the value of *Q* is used as *Q'* in an exemplary embodiment. More aggressiveness is not warranted in this latter case as the AID system has less confidence in the glucose level value provided by the CGM.

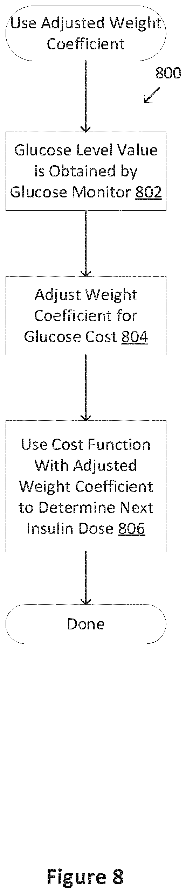

Figure 8 depicts a flowchart 800 of illustrative steps that may be performed in exemplary embodiments to adjust the glucose cost weight coefficient based on the robustness factor. At 802, a glucose level value is obtained from a sensor 106, like a CGM. In some circumstances (e.g., when the robustness factor $R_f(t)$ is greater than 1), at 804, the glucose cost weight coefficient may be adjusted. At 806, the cost function with the adjusted glucose cost component weight may be used by the AID controller to determine the next basal insulin dose for the user 108.

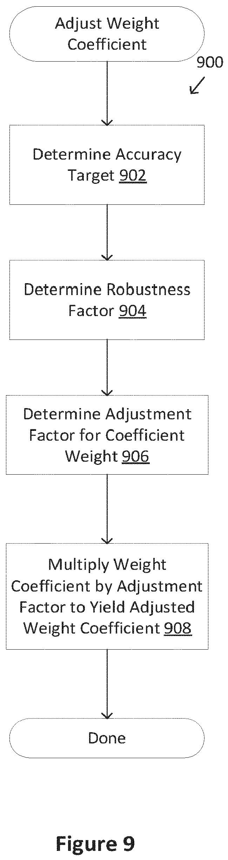

Figure 9 depicts a flowchart 900 of illustrative steps that may be performed in exemplary embodiments to adjust the glucose cost weight coefficient. At 902, the accuracy target $A_{target}$ may be determined, such as described above relative to Figure 5. At 904, the robustness factor $R_f(t)$ may be determined, such as described above relative to Figure 6. At 906, an adjustment factor for the glucose cost weight coefficient may be determined.

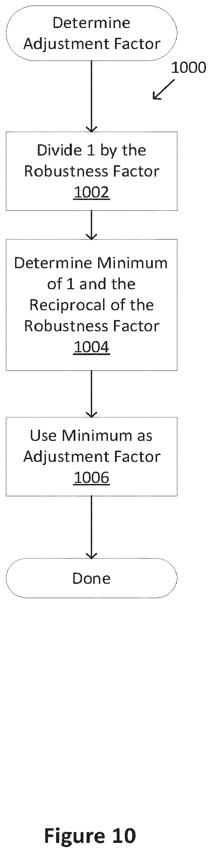

Figure 10 depicts a flowchart 1000 of illustrative steps that may be performed in exemplary embodiments to determine the adjustment factor (see 906). At 1002, 1 is divided by the robustness factor $R_f(t)$ to calculate the reciprocal of the robustness factor. At 1004, the minimum of 1 and the reciprocal of the robustness factor is determined. At 1006, the minimum may be used as the adjustment factor. This may be summarized in equation form as:

$$min \left( 1, \frac{1}{R_f(t)} \right) \text{ (Equation 5)}.$$

or

$$adjustment\ factor = min \left( 1, \frac{1}{R_f(t)} \right) \text{ (Equation 5a)}.$$

With reference to Figure 9 again, at 908, the weight coefficient for the glucose cost may be multiplied by the adjustment factor to produce the adjusted weight coefficient. The adjusted weight coefficient may be expressed as:

$$Q' = Q \cdot min \left( 1, \frac{1}{R_f(t)} \right) \text{ (Equation 6)}.$$

Hence, the weight coefficient for the glucose cost component is either the existing coefficient Q or a version of *Q* that has been adjusted by the reciprocal of the robustness factor $R_f(t)$.

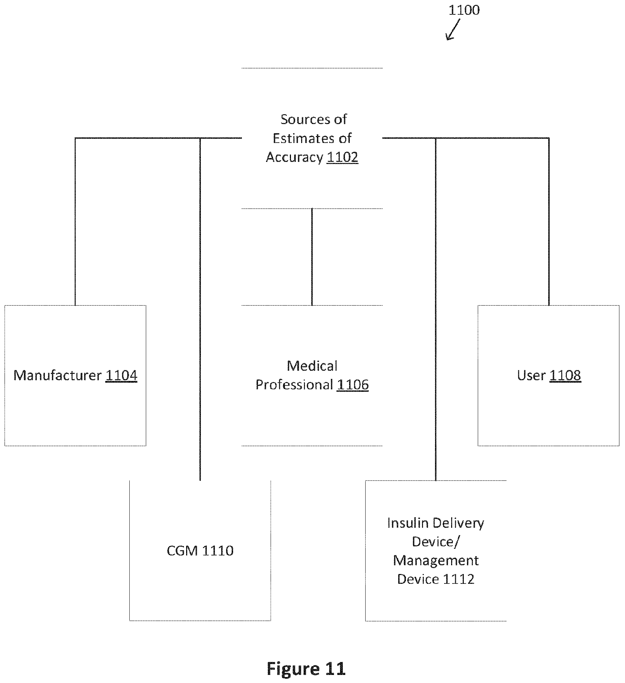

The above-described approaches to compensate for sensor value inaccuracies rely on estimates of the magnitude of the inaccuracies. These estimates may be provided by different sources. Figure 11 depicts a diagram 1100 identifying some illustrative sources 1102. The estimate may originate from the manufacturer 1104 of the sensor. For example, the manufacturer may publish MARD values for the sensor over a period of time (e.g., during the period of use, such as 10 days) derived from trials or other empirical data sources. A medical professional 1106 may provide an estimate of the sensor inaccuracy. This may be based on the experience to date on working with patients that use the sensor. A user 1108 may originate the estimate based on their personal experience or by comparing values to more accurate values from another sensor. Where the sensor is a CGM, the CGM 1112 may be the source, and may include logic for estimating or conveying the estimate of the magnitude of inaccuracies. Where the medicament delivery device 102 is an insulin delivery device, the insulin delivery device or management device 1112 may originate the estimate based on logic contained therein or by comparing the expected glucose levels with the actual glucose levels. In some cases, the magnitude of the inaccuracies may vary over time (e.g., tested and published MARD values by a CGM manufacturer); in other cases, the magnitude of the inaccuracies may be used as a constant. In some cases, the magnitude of the inaccuracies may be estimated based on the user's blood glucose level history and a related use duration of a sensor. For example, the medicament delivery system may analyze the user's blood glucose level history and determine that on average the sensor provides sensor values that are 10 % lower during its first 4 hours of use. The blood glucose level history of the user may be adjusted for other factors, such as consumed meals.

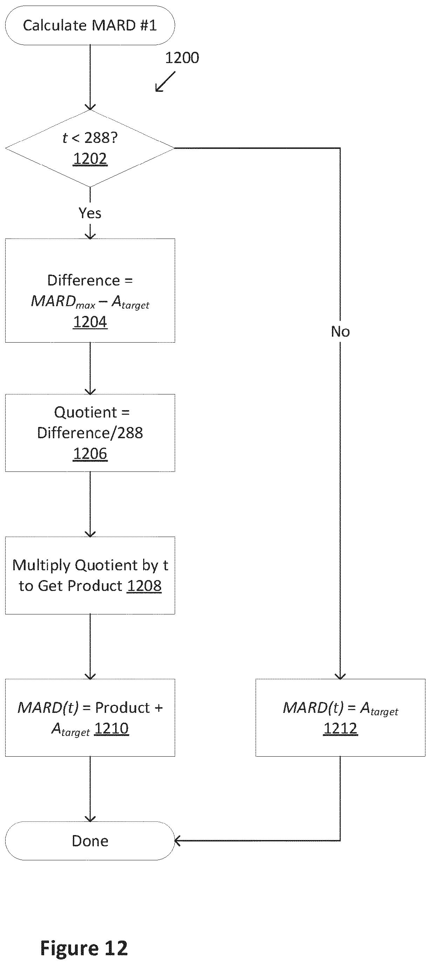

As mentioned above, in some exemplary embodiments, the MARD values may be calculated. Figure 12 depicts a flowchart 1200 of illustrative steps that may be performed in exemplary embodiments in accordance with a first approach. At 1202, a check is made whether the current cycle number $t$ has a value of less than 288. In this instance, the cycles are 5 minutes in length. Hence, there are 288 cycles in a day. The check at 1202, determines whether the cycle is not part of the first day. With CGMs, the accuracy typically varies between day 1 and days 2-10 of the time that the CGM is secured to the user 108. If the cycle number $t$ is not less than 288, at 1212, $MARD(t)$ is set as $A_{target}$, where $A_{target}$ may be used to reflect the expected accuracy of the CGM If $t$ is less than or equal to 288, at 1204, a difference between $MARD_{max}$ and $A_{target}$ is calculated. $MARD_{max}$ may be the best case accuracy of the CGM or the ending accuracy of the CGM. Hence, the difference represents how much $MARD_{max}$ exceeds the target of $A_{target}$. At 1206, a quotient of the difference divided by 288 is calculated, and at 1208, the quotient is multiplied by the current cycle number $t$. Thus, the net effect of 1206 and 1208 is to multiply the difference by $t/288$, which represents a portion of the day that has passed. At 1210, the product is added to $A_{target}$ to determine $MARD(t)$.

The first option may be summarized as determining $MARD(t)$ as follows:

$$MARD(t) = \begin{cases} t < 288 & \frac{MARD_{max} - A_{target}}{288} \cdot t + A_{target} \\ t \geq 288 & A_{target} \end{cases} \text{ (Equation 7)}.$$

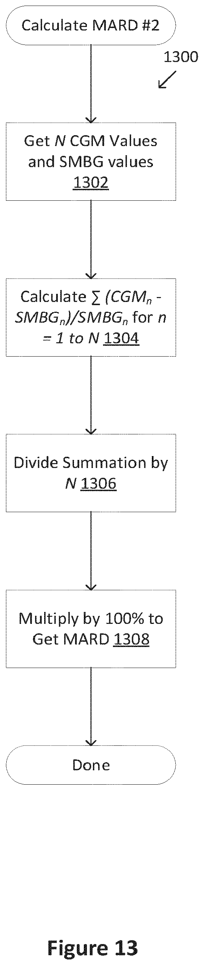

Figure 13 depicts a flowchart 1300 of illustrative steps that may be performed in exemplary embodiments in accordance with a second approach for determining $MARD(t)$. In this second approach, $MARD(t)$ is calculated with the assistance of glucose level values from self-monitored blood glucose (SMBG) values, such as derived by using blood sugar monitors where a blood sample is obtained by a finger prick and processed by the monitor to obtain a blood glucose level. Such monitors are known to be more accurate than CGMs. At 1302, $N$ CGM values and $N$ SMBG values are obtained for successive cycles. At 1304, a summation of the differences between $CGM_n$ and $SMBG_n$ divided by $SMBG_n$ is calculated. At 1306, the summation is divided by $N$, where $N$ is the number of cycles over which the summation is performed. The resulting quotient represents the average ratio of the differences of the CGM values to the SMBG values. At 1308, the resulting average ratio is multiplied by 100 to yield a percentage value for the MARD($t$) for the $N$ cycles. The calculation may be captured by the equation:

$$MARD \% = \frac{1}{N} \sum_{n=1}^{N} ((|CGM_n - SMBG_n|)/SMBG_n) * 100 \text{ (Equation 8)}.$$

While exemplary embodiments have been described herein, various changes in from and detail may be made without departing from the intended scope of the claims appended hereto. The methods described herein may be available in the form of computer programs. The computer programs of the present disclosure may be for example preinstalled on, or downloaded to the medicament delivery system, medicament delivery device, management device, fluid delivery device, e.g. their storage.

Although the present invention is defined in the attached claims, it should be understood that the present invention can also (alternatively) be defined in accordance with the following embodiments:

1. A medicament delivery device for delivering a medicament to a user, comprising:

a non-transitory computer-readable storage medium storing computer programming instructions;
a processor configured to execute the computer programming instructions to cause the processor to:

adjust a received sensor value from a sensor for an interval in a period based on an accuracy estimate for the glucose sensor;
use the adjusted received sensor value to determine a dose of medicament to be delivered to the user for the interval; and
cause the determined dose of the medicament to be delivered by the medicament delivery device to the user.

2. The medicament delivery device of embodiment 1, wherein the accuracy estimate is based at least in part on a manufacturer accuracy estimate for the sensor.

3. The medicament delivery device of embodiment 1, wherein executing the computer programming instructions further causes the processor to determine the accuracy estimate.

4. The medicament delivery device of embodiment 1, wherein the computer programming instructions further cause the processor to:

determine an accuracy target for the sensor values from the sensor as an average of expected accuracy of the sensor values over a period;
determine a robustness factor for the sensor values for the interval in the period as a quotient of the determined accuracy target divided by the expected accuracy of the senor values for the interval of the period; and
wherein the determined robustness factor is the accuracy estimate

5. The medicament delivery device of embodiment 4, wherein the adjusting of the received sensor value comprises multiplying the received sensor value by the robustness factor if the robustness factor is less than one.

6. The medicament delivery device of embodiment 5, wherein the adjusting of the received sensor value comprises multiplying the received sensor value by one if the robustness factor is greater than or equal to one.

7. The medicament delivery device of embodiment 4, wherein the expected accuracy of the sensor values of the period is a mean absolute relative difference (MARD) value.

8. The medicament delivery device of embodiment 1, wherein the sensor is a glucose monitor.

9. The medicament delivery device of embodiment 8, wherein the glucose monitor is a continuous glucose monitor (CGM).

10. The medicament delivery device of embodiment 1, wherein the medicament comprises insulin.

11. The medicament delivery device of embodiment 1, wherein the medicament delivery device has a lifespan and wherein the period is the lifespan.

12. A medicament delivery device for delivering medicament to a user, comprising:

a non-transitory computer-readable storage medium storing computer programming instructions;
a processor configured to execute the computer programming instructions to cause the processor to:

determine an accuracy target for the sensor values from a sensor as an average of expected accuracy of the sensor values over a period;
determine a robustness factor for the sensor values for an interval of the period as a quotient of the determined accuracy target divided by an expected accuracy of a sensor value for the interval;
adjust a weight coefficient for a cost component of a cost function based on the robustness factor, wherein the cost component reflects a cost of a difference between a target value and a sensor value for a current interval;
use the cost function with the adjusted weight coefficient to determine a dose of the medicament for the current interval; and
cause the determined dose of the medicament to be delivered by the medicament delivery device to the user.

13. The medicament delivery device of embodiment 12, wherein the medicament comprises insulin.

14. The medicament delivery device of embodiment 13, wherein the weight coefficient is for a glucose cost component that reflects a difference between a target glucose level for the user and a glucose level reading for the current interval.

15. The medicament delivery device of embodiment 14, wherein the cost function also includes an insulin cost component reflective of how a candidate dose of insulin exceeds a target dose.

16. The medicament delivery device of embodiment 12, wherein the period includes multiple days.

17. The medicament delivery device of embodiment 12, wherein the interval is an operational cycle of the medicament delivery device and wherein a dose of the medicament to be delivered is calculated for each operational cycle while the medicament delivery device is in use by the user.

18. An on-body glucose sensor attached to a user for sensing a glucose level of a user, comprising;

a sensing element positioned under skin of the user to sense the glucose level values of the user; and
a filter for filtering the sensed glucose level values to modify the glucose level values based on how long the glucose sensor has been attached to the user to compensate for inaccuracy of the glucose sensor.

19. The on-body glucose sensor of embodiment 18, wherein the filter is a dynamic filter that modifies the filtering over time.

20. The on-body glucose monitor of embodiment 18, wherein the filter is a low pass filter.

21. An electronic device, comprising:

a non-transitory computer-readable storage medium storing computer programming instructions;
a processor configured to execute the computer programming instructions to cause the processor to:

determine an accuracy target for sensor values from a sensor over a period as an average of expected accuracy of the sensor values over a period;
determine a robustness factor for the sensor values for an interval in the period as a quotient of the determined accuracy target divided by an expected accuracy of a sensor value for the interval;
adjust a sensor value generated by the sensor for the interval based on the determined robustness factor; and
forward the adjusted sensor value to a medicament delivery device for use in determining a dose of medicament to be delivered to a user.

22. The electronic device of embodiment 21, wherein the electronic device is one of a glucose monitor or a management device for the medicament delivery device.

23. The electronic device of embodiment 21, wherein the medicament is insulin.

## Claims

1. A medicament delivery system for delivering a medicament to a user, comprising:

a non-transitory computer-readable storage medium storing computer programming instructions;
a processor configured to execute the computer programming instructions to cause the processor to:

adjust a received sensor value from a sensor for an interval in a period based on an accuracy estimate for the glucose sensor;
use the adjusted received sensor value to determine a dose of medicament to be delivered to the user for the interval; and

cause the determined dose of the medicament to be delivered by the medicament delivery system to the user.

2. The medicament delivery system of claim 1, wherein the accuracy estimate is based at least in part on a manufacturer accuracy estimate for the sensor.

3. The medicament delivery system of claim 1 or 2, wherein executing the computer programming instructions further causes the processor to determine the accuracy estimate.

4. The medicament delivery system of any one of claims 1 to 3, wherein the computer programming instructions further cause the processor to:

determine an accuracy target for the sensor values from the sensor as an average of expected accuracy of the sensor values over a period;
determine a robustness factor for the sensor values for the interval in the period as a quotient of the determined accuracy target divided by the expected accuracy of the senor values for the interval of the period; and
wherein the determined robustness factor is the accuracy estimate

5. The medicament delivery system of claim 4, wherein the adjusting of the received sensor value comprises multiplying the received sensor value by the robustness factor if the robustness factor is less than one, and/or
wherein the adjusting of the received sensor value comprises multiplying the received sensor value by one if the robustness factor is greater than or equal to one.

6. The medicament delivery system of any one of claims 4 to 5, wherein the expected accuracy of the sensor values of the period is a mean absolute relative difference (MARD) value.

7. The medicament delivery system of any one of claims 1 to 6, wherein the sensor is a glucose monitor, in particular a continuous glucose monitor (CGM).

8. The medicament delivery system of claim 1, wherein the medicament delivery system, and/or a medicament delivery device comprised in the medicament delivery system, has a lifespan and wherein the period is the lifespan.

9. A medicament delivery system for delivering medicament to a user, comprising:

a non-transitory computer-readable storage medium storing computer programming instructions;
a processor configured to execute the computer programming instructions to cause the processor to:

determine an accuracy target for the sensor values from a sensor as an average of expected accuracy of the sensor values over a period;
determine a robustness factor for the sensor values for an interval of the period as a quotient of the determined accuracy target divided by an expected accuracy of a sensor value for the interval;
adjust a weight coefficient for a cost component of a cost function based on the robustness factor, wherein the cost component reflects a cost of a difference between a target value and a sensor value for a current interval;
use the cost function with the adjusted weight coefficient to determine a dose of the medicament for the current interval; and
cause the determined dose of the medicament to be delivered by the medicament delivery system to the user.

10. The medicament delivery system of claim 9, wherein the weight coefficient is for a glucose cost component that reflects a difference between a target glucose level for the user and a glucose level reading for the current interval, in particular wherein the cost function also includes an insulin cost component reflective of how a candidate dose of insulin exceeds a target dose.

11. The medicament delivery system of claim 9, wherein the period includes multiple days.

12. The medicament delivery system of claim 9, wherein the interval is an operational cycle of the medicament delivery system, or a medicament deliver device comprised in the medicament delivery system, and wherein a dose of the medicament to be delivered is calculated for each operational cycle while the medicament delivery system, or medicament delivery device, is in use by the user.

13. An on-body glucose sensor attached to a user for sensing a glucose level of a user, comprising;

   a sensing element positioned under skin of the user to sense the glucose level values of the user; and
   a filter for filtering the sensed glucose level values to modify the glucose level values based on how long the glucose sensor has been attached to the user to compensate for inaccuracy of the glucose sensor.

14. The on-body glucose sensor of claim 13, wherein the filter is a dynamic filter that modifies the filtering over time and/or wherein the filter is a low pass filter.

15. An electronic device, comprising:

   a non-transitory computer-readable storage medium storing computer programming instructions;
   a processor configured to execute the computer programming instructions to cause the processor to:

      determine an accuracy target for sensor values from a sensor over a period as an average of expected accuracy of the sensor values over a period;
      determine a robustness factor for the sensor values for an interval in the period as a quotient of the determined accuracy target divided by an expected accuracy of a sensor value for the interval;
      adjust a sensor value generated by the sensor for the interval based on the determined robustness factor; and
      forward the adjusted sensor value to a medicament delivery device or medicament delivery system for use in determining a dose of medicament to be delivered to a user.

Figure 1

Adjust Sensor Value

200

Sensor Value is
Sensed by Sensor
202

Adjust Received
Sensor Value for
Accuracy 204

Use Adjusted Sensor
Value in Dose
Determination 206

Done

**Figure 2A**

Time Trigger

208

Trigger Time is
Reached 210

Perform
Adjustment of
Sensor Value 212

Done

**Figure 2B**

Event Trigger

220

Trigger Event is
Detected 222

Perform
Adjustment of
Sensor Value 224

Done

**Figure 2C**

Adjusted
Glucose Level
Value 308

AID Control 310

Insulin Delivery
Device 304

Adjust 306

Unadjusted Glucose
Level Value 302

CGM 300

Figure 3A

| AID Control 310 |
| --- |
| Insulin Delivery Device 304 |

← Adjusted Glucose Level Value 308 ←

| CGM 300 |
| --- |
| Detector 312 |
| Adjust 316 |

| Unadjusted Glucose Level Value 302 |
| --- |

**Figure 3B**

Unadjusted Glucose Level Value 302

Detector 312

CGM 300

Adjust 322

Management Device 320

Adjusted Glucose Level Value 308

AID Control 310

Insulin Delivery Device 304

Figure 3C

Adjust Glucose
Value

400

Determine
Accuracy Target
402

Determine
Robustness
Factor 404

Adjust Received
Glucose Level
Value 406

Done

Figure 4

```
          ╭─────────────────────╮
          │  Determine Accuracy │
          │       Target        │
          ╰─────────────────────╯
                     │            500
                     ▼           ↙
          ┌─────────────────────┐
          │  Obtain Estimates of │
          │ Accuracy Over Lifetime│
          │     That Device is   │
          │    Attached 502      │
          └─────────────────────┘
                     │
                     ▼
          ┌─────────────────────┐
          │   Sum Estimates for  │
          │ Days Attached to Date│
          │        504           │
          └─────────────────────┘
                     │
                     ▼
          ┌─────────────────────┐
          │  Divide Sum by Number│
          │   of Days Attached to│
          │  Date to get an Average│
          │        506           │
          └─────────────────────┘
                     │
                     ▼
          ┌─────────────────────┐
          │     Use Average as   │
          │  Accuracy Target 508 │
          └─────────────────────┘
                     │
                     ▼
          ╭─────────────────────╮
          │        Done         │
          ╰─────────────────────╯
```

# Figure 5

```
        ╭─────────────────╮
        │    Determine    │
        │ Robustness Factor │
        ╰─────────────────╯
                 │              600
                 │         ↙
                 ▼
        ┌─────────────────┐
        │  Divide Accuracy │
        │ Target by Estimate │
        │  for Cycle to get a │
        │  Quotient 602   │
        └─────────────────┘
                 │
                 │
                 ▼
        ┌─────────────────┐
        │  Set Quotient as │
        │ Robustness Factor │
        │  for Cycle 604  │
        └─────────────────┘
                 │
                 │
                 ▼
        ╭─────────────────╮
        │      Done       │
        ╰─────────────────╯
```

# Figure 6

Adjust Glucose Level Value

700

Obtain Unadjusted Glucose Level Value for Cycle From the Detector of the Glucose Monitor 702

Determine Adjustment Factor as Minimum of 1 and Robustness Factor for Cycle 704

Multiply Unadjusted Glucose Level Value By Adjustment Factor to Get Adjusted Glucose Level Value 706

Done

**Figure 7**

Use Adjusted Weight Coefficient

800

Glucose Level Value is Obtained by Glucose Monitor 802

Adjust Weight Coefficient for Glucose Cost 804

Use Cost Function With Adjusted Weight Coefficient to Determine Next Insulin Dose 806

Done

Figure 8

Adjust Weight
Coefficient

900

Determine Accuracy
Target 902

Determine Robustness
Factor 904

Determine Adjustment
Factor for Coefficient
Weight 906

Multiply Weight
Coefficient by Adjustment
Factor to Yield Adjusted
Weight Coefficient 908

Done

**Figure 9**

Determine
Adjustment Factor

1000

Divide 1 by the
Robustness Factor
1002

Determine Minimum
of 1 and the
Reciprocal of the
Robustness Factor
1004

Use Minimum as
Adjustment Factor
1006

Done

**Figure 10**

1100

Sources of
Estimates of
Accuracy 1102

Manufacturer 1104

Medical
Professional 1106

User 1108

CGM 1110

Insulin Delivery
Device/
Management
Device 1112

Figure 11

EP 4 390 953 A1

Calculate MARD #1

1200

t < 288?
1202

Yes

Difference =
$MARD_{max} - A_{target}$
1204

Quotient =
Difference/288
1206

Multiply Quotient by t
to Get Product 1208

$MARD(t)$ = Product +
$A_{target}$ 1210

No

$MARD(t) = A_{target}$
1212

Done

Figure 12

Calculate MARD #2

1300

Get *N* CGM Values
and SMBG values
1302

Calculate $\sum (CGM_n - SMBG_n)/SMBG_n$ for *n = 1 to N* 1304

Divide Summation by
*N* 1306

Multiply by 100% to
Get MARD 1308

Done

Figure 13

Europäisches
Patentamt

European
Patent Office

Office européen
des brevets

## PARTIAL EUROPEAN SEARCH REPORT

under Rule 62a and/or 63 of the European Patent Convention.
This report shall be considered, for the purposes of
subsequent proceedings, as the European search report

**Application Number**

**EP 23 21 8097**

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/386335 A1 (VARSAVSKY ANDREA [US] ET AL) 16 December 2021 (2021-12-16) | 1-3,7,8 | INV.<br>G16H20/17 |
| A | * paragraphs [0002], [0025], [0029], [0145], [0281], [0285] * | 4-6 | G16H40/63<br>A61M5/172 |
| A | FÁTIMA BARCELÓ-RICO ET AL: "A Multiple Local Models Approach to Accuracy Improvement in Continuous Glucose Monitoring", DIABETES TECHNOLOGY & THERAPEUTICS, vol. 14, no. 1, 1 January 2012 (2012-01-01), pages 74-82, XP055073754, ISSN: 1520-9156, DOI: 10.1089/dia.2011.0138 * abstract * | 1-8 | |

**TECHNICAL FIELDS SEARCHED (IPC)**

G16H

### INCOMPLETE SEARCH

The Search Division considers that the present application, or one or more of its claims, does/do not comply with the EPC so that only a partial search (R.62a, 63) has been carried out.

Claims searched completely :

Claims searched incompletely :

Claims not searched :

Reason for the limitation of the search:

**see sheet C**

| Place of search | Date of completion of the search | Examiner |
|---|---|---|
| Munich | 15 May 2024 | Reinbold, Bernhard |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
........................................................................
& : member of the same patent family, corresponding
   document

EPO FORM 1503 03.82 (P04E07)

Europäisches
Patentamt

European
Patent Office

Office européen
des brevets

**INCOMPLETE SEARCH
SHEET C**

```
Claim(s) completely searchable:
     1-8

Claim(s) not searched:
     9-15

Reason for the limitation of the search:

The set of claims as originally filed comprises four independent system
claims (claims 1, 9, 13 and 15). These claims do not comply with Rule
43(2) EPC. Therefore, on 30-04-2024 an invitation pursuant to Rule 62a(1)
EPC was sent to the applicant. In the reply received on 10-05-2024 the
applicant indicated claim 1 as basis of the search. In conclusion, claim
1 and its dependent claims, i.e. claims 2 to 8, have been searched.
```

## ANNEX TO THE EUROPEAN SEARCH REPORT
## ON EUROPEAN PATENT APPLICATION NO.

EP 23 21 8097

This annex lists the patent family members relating to the patent documents cited in the above-mentioned European search report.
The members are as contained in the European Patent Office EDP file on
The European Patent Office is in no way liable for these particulars which are merely given for the purpose of information.

15-05-2024

| Patent document cited in search report | Publication date | Patent family member(s) | Publication date |
|---|---|---|---|
| US 2021386335 A1 | 16-12-2021 | AU 2014367039 A1 | 16-06-2016 |
| | | AU 2019226130 A1 | 26-09-2019 |
| | | CA 2931955 A1 | 25-06-2015 |
| | | CA 3111452 A1 | 25-06-2015 |
| | | CN 106028932 A | 12-10-2016 |
| | | CN 109984753 A | 09-07-2019 |
| | | CN 109984754 A | 09-07-2019 |
| | | CN 109984755 A | 09-07-2019 |
| | | EP 3082597 A2 | 26-10-2016 |
| | | EP 3733065 A1 | 04-11-2020 |
| | | JP 6339204 B2 | 06-06-2018 |
| | | JP 6502552 B2 | 17-04-2019 |
| | | JP 6717997 B2 | 08-07-2020 |
| | | JP 2017500942 A | 12-01-2017 |
| | | JP 2018149327 A | 27-09-2018 |
| | | JP 2019115714 A | 18-07-2019 |
| | | KR 20160098487 A | 18-08-2016 |
| | | US 2015164383 A1 | 18-06-2015 |
| | | US 2015164384 A1 | 18-06-2015 |
| | | US 2015164385 A1 | 18-06-2015 |
| | | US 2015164388 A1 | 18-06-2015 |
| | | US 2015164389 A1 | 18-06-2015 |
| | | US 2017150911 A1 | 01-06-2017 |
| | | US 2017209080 A1 | 27-07-2017 |
| | | US 2017209082 A1 | 27-07-2017 |
| | | US 2018184951 A1 | 05-07-2018 |
| | | US 2019167170 A1 | 06-06-2019 |
| | | US 2019343434 A1 | 14-11-2019 |
| | | US 2019357820 A1 | 28-11-2019 |
| | | US 2021161439 A1 | 03-06-2021 |
| | | US 2021386335 A1 | 16-12-2021 |
| | | US 2022354394 A1 | 10-11-2022 |
| | | WO 2015094576 A2 | 25-06-2015 |

EPO FORM P0459

For more details about this annex : see Official Journal of the European Patent Office, No. 12/82